(19) Europäisches Patentamt
European Patent Office
Office européen des brevets

(11) Publication number: 0 350 318 A2

(12) # EUROPEAN PATENT APPLICATION

(21) Application number: 89306902.1

(22) Date of filing: 06.07.89

(51) Int. Cl.$^5$: C 07 K 7/00
A 61 K 37/02

(30) Priority: 07.07.88 DK 3802/88

(43) Date of publication of application:
10.01.90 Bulletin 90/02

(84) Designated Contracting States:
AT BE CH DE ES FR GB GR IT LI LU NL SE

(71) Applicant: NOVO-NORDISK A/S
Novo Allé
DK-2880 Bagsvaerd (DK)

(72) Inventor: Johansen, Nils Langeland
Nils W. Gades Gade 45
DK-2100 Copenhagen (DK)

Thogersen, Henning
Kaerbovaenge 26
DK-3520 Farum (DK)

Faarup, Peter
Skraplanet 15 Jonstrup
DK-3500 Vaerlose (DK)

Lundt, Behrend Friedrich
Rosenhaven 18 Bronsholm
DK-2980 Kokkedal (DK)

Weis, Jan Ulrik
Fuglsangvej 6
DK-2830 Virum (DK)

(74) Representative: Brown, John David et al
FORRESTER & BOEHMERT Widenmayerstrasse 4/I
D-8000 München 22 (DE)

(54) **Novel peptides.**

(57) Analogues to atrial natriuretic peptides (ANP) containing only the ring structure have improved pharmacological properties.

EP 0 350 318 A2

**Description**

## NOVEL PEPTIDES

FIELD OF THE INVENTION

The present invention relates to novel synthetic analogues of atrial peptides having interesting and useful pharmacological effects such as natriuretic, diuretic and vasodilating activity, to the use of such peptides as medicaments, for example in the treatment of hypertension, congestive heart failure and diseases in the renal function, and to a method for their preparation.

BACKGROUND ART

Throughout the present specification the term amino acid (or amino acid residue), unless otherwise specified, refers to one of the α-amino acids which are listed below together with the symbols used to designate the individual amino acid residues:

| | | | |
|---|---|---|---|
| Asp | Aspartic acid | Ile | Isoleucine |
| Thr | Threonine | Leu | Leucine |
| Ser | Serine | Tyr | Tyrosine |
| hSer | Homo-serine | Phe | Phenyla-lanine |
| Glu | Glutamic acid | His | Histidine |
| Pro | Proline | Lys | Lysine |
| Gly | Glycine | Arg | Arginine |
| Sar | Sarcosine | hArg | Homoar-ginine |
| Ala | Alanine | Trp | Trypto-phane |
| Cys | Cysteine | Gln | Glutamine |
| Val | Valine | Asn | As-paragine |
| Met | Meth-ionine | | |

Ac designates an acetyl group. Thus for example Ac-Gly-OH designates $N^{\alpha}$-acetylglycine. It is known from the pioneering observation (Life Sciences 28 (1981), 89 - 94), that extracts of atrial myocardium when injected intravenously into non-diuretic rats provoke a rapid and potent diuretic and natriuretic response. This natriuretic and diuretic factor of atrial origin has later on been purified both from the atrial muscle of the rat and from other mammals. The factor is now known to be a polypeptide which has been characterized in terms of the amino acid sequence. The polypeptide comprises a 17 amino acid section which is closed to a ring by a disulfide bridge formed by two cystein residues and the peptide chain continues through both the N-terminus and the C-terminus of said cyclic section. Experimental evidence suggests that a 28 amino acid peptide is released from the heart and circulates in the body in both rats and humans (Biochem.Biophys.Res.Comm. 129 (1985), 439 - 446; and Science 229 (1985), 397 - 400). The primary structure of the 28 amino acid peptide from rat is shown in formula (I):

```
H-Ser-Leu-Arg-Arg-Ser-Ser-Cys-Phe-Gly-Gly-Arg-Ile-Asp-Arg
                    5      |                10           |
                           S                             |
                           |                       15 Ile
                           S                             |
                           |                             |
   HO-Tyr-Arg-Phe-Ser-Asn-Cys-Gly-Leu-Gly-Ser-Gln-Ala-Gly
                     25                 20
```

(I)

The sequence of the corresponding human peptide differs by only one amino acid residue, in that the human

peptide has Met in position 12 instead of Ile.

Hereinafter, the rat atrial natriuretic peptide will be referred to by the abbreviation ANP and numerals will be used to indicate specific sequences with ANP(1-28) (numbered from the N- to the C-terminus) serving as the reference polypeptide. Hence, ANP(5-28) is ANP from which -$Ser^1$-$Leu^2$-$Arg^3$-$Arg^4$- is omitted. [$Leu^{18}$]-ANP is ANP wherein $Gln^{18}$ is substituted by Leu. The abbreviation hANP is the human analog to ANP, i.e. ANP wherein $Ile^{12}$ is substituted by Met. In some compounds according to this invention the ring consists entirely of amino acid residues linked together by peptide bonds. These compounds can be described with reference to ANP as stated above using a first element of the form "cyclo(X,Y)", which designates that the amino group of amino acid residue No. X is linked through a peptide bond to the carboxyl group of amino acid residue No. Y. Alternatively, the whole sequence of amino acid residues can be stated and a horizontal bracket be used to indicate the cyclization. Unless otherwise indicated, the amino acids have the L-configuration.

Over the last decades a variety of polypeptide drugs have been developed. In general, polypeptides have been administered parenterally due to incomplete absorption from the digestive instability in the alimentary canal. However, a non-invasive medication is more convenient to the patient and therefore studies of transmucosal and particularly of nasal delivery have been intensified during recent years. From these studies it has emerged that the nasal bioavailability of larger polypeptides is incomplete and variable, and increasingly so with increasing molecular weight, while smaller polypeptides may be reasonably well absorbed intranasally.

Several derivatives of ANP and hANP are known. However no truncated ANP or hANP derivatives are known in which the amino acid residues 1 - 6 and 24 - 28 or 1-7 and 23-28 are omitted and in which referring to formula I two basic amino acid residues are positioned between $Ile^{15}$ and $Leu^{21}$ especially in combination with the exchange of $Leu^{21}$ with an amino acid residue with an aromatic side chain.

DISCLOSURE OF THIS INVENTION

Thus, according to a first aspect of the present invention, novel polypeptide compounds with ANP-like natriuretic and vasodilator properties are provided for the regulation of fluid volume and blood pressure in host organisms.

According to a second aspect of the present invention novel polypeptide compounds with ANP-like natriuretic and vasodilator properties are provided free from impurities of similar origin as native ANP.

According to a third aspect of the present invention novel polypeptide compounds with ANP-like natriuretic and vasodilator properties are provided which compounds comprise fewer amino acid residues than ANP (1-28) yet have improved pharmacological properties when compared to the truncated derivative [Ac-$Cys^7$,$Cys^{23}$-$NH_2$]-ANP(7-23).

It has turned out that in order to obtain ANP-analogues having the desired pharmacological activity there are rather specific structural requirements to be met in certain regions of the molecule while the structural requirements in other regions of the molecule are less rigorous.

Thus the present invention relates to novel cyclic compounds of the general formula II

$$\overbrace{A^1-X^1-A^2-A^3-A^4-X^2-A^5-X^3-A^6-A^7-X^4}^{B}$$

(II)

wherein the symbols $A^1$, $A^2$, $A^3$, $A^4$, $A^5$, $A^6$ and $A^7$ designate substructures (amino acids residues) of rather specific kind while the symbols $X^1$, $X^2$, $X^3$ and $X^4$ designate substructures (bonds, amino acid residues or peptides residues) of less specific kind. The substructure B can be of an even more varied nature.

It is believed that the substructures $X^1$, $X^2$, $X^3$, $X^4$ and B mainly serve as a kind of spacers, which keep the active substructures $A^1$, $A^2$, $A^3$, $A^4$, $A^5$, $A^6$ and $A^7$ in a well defined position relative to each other.

In formula II

$A^1$ is Phe or D-Phe, preferably Phe;

$A^2$ is Asp, D-Asp, Glu or Gly, preferably Asp;

$A^3$ is Arg or hArg, preferably Arg;

$A^4$ is Ile, Leu or Val, preferably Ile;

$A^5$ is Arg, D-Arg, hArg, another amino acid residue with a basic side chain or Ala, preferably Arg;

$A^6$ is D-Arg, Arg or another amino acid residue in the D-or L-configuration with a basic side chain, preferably D-Arg;

$A^7$ is Leu, Phe or another amino acid residue with an aromatic side chain, preferably Phe;

$X^1$ is a bond or a moiety of the formula

$$-(A^a)_{n_1}-(A^b)_{n_2}-(A^c)_{n_3}-(A^d)_{n_4}-$$

where $A^a$, $A^b$, $A^c$ and $A^d$ each independently is an amino acid residue in the D- or L-configuration with a neutral or a basic side chain other than the sarcosine residue and $n_1$, $n_2$, $n_3$ and $n_4$ independently are zero or one; preferably $A^a$ is an amino acid residue with a neutral side chain, more preferred $A^a$ is Gly, D-Ala or D-Phe, $A^b$ is preferably Gly, $A^c$ is preferably Arg and $A^d$ is preferably Ile and $n_1$, $n_2$, $n_3$ and $n_4$ are preferably one;
$X^2$ is a bond or a moiety of the formula $-(A^e)_{n_5}-$ where $A^e$ is an amino acid residue with a neutral side chain other than the sarcosine residue and $n_5$ is zero or one, preferably $A^e$ is Ala or Gly;
$X^3$ is a bond or a moiety of the formula

$$-(A^f)_{n_6}-(A^g)_{n_7}-$$

where $A^f$ and $A^g$ each independently is an amino acid residue with a neutral side chain other than the sarcosine residue and $n_6$ and $n_7$ independently are zero or one; preferably $A^f$ is Gln, Leu, Ala or Gly, and preferably $A^g$ is Ser, Ala or Gly, more preferred $A^g$ is Ser;
$X^4$ is a bond or a moiety of the formula

$$-(A^h)_{n_8}-(A^k)_{n_9}-$$

where $A^h$ and $A^k$ each independently is an amino acid residue in the D- or L-configuration with a neutral side chain or in the case of $A^h$: 8-aminooctanoic acid and $n_8$ and $n_9$ each independently are zero or one; preferrably $A^h$ is Gly, D-Ala, Ala, Sar or 8-aminooctanoic acid, and preferably $n_9$ is zero;
B is either a peptide bond connecting the N-terminus of $A^1$ with the C-terminus of $X^4$ or a substructure of the general formula III

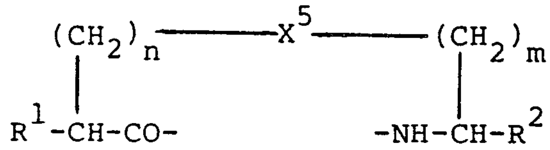

(III)

which is oriented so that the -CO- group is linked through a peptide bond to the N-terminus of $A^1$ and the -NH- group is linked through a peptide bond to the C-terminus of $X^4$ and in which
$R^1$ is hydrogen, amino or lower acylamido, preferably hydrogen or acetamido;
$R^2$ is hydrogen, carboxyl or carbamoyl, preferably carbamoyl;
$X^5$ is a carbon-carbon bond or a group selected from -S-S-, -CO-NH- or -NH-CO-, preferably $X^5$ is -S-S-;
n and m are independently 0, 1, 2, 3 or 4 with the proviso that when B is different from just a peptide bond the total number of chain members in the part of B which connects the N-terminus of $A^1$ with the C-terminus of $X^4$ is from 4 to 9; preferably n and m are both one.

In the definition of $R^1$ lower acyl designates an acyl group containing up to 7 carbon atoms, preferably up to 5 carbon atoms, for example pivaloyl, butyryl, propionyl, acetyl and formyl. More preferred the acyl group is acetyl.

Amino acid residues (D- or L-) with a basic side chain are selected from the following group: Arg, hArg, His and Lys.

Amino acid residues (D- or L) with an aromatic side chain are selected from the following group: Phe, Tyr and Trp.

Amino acid residues (D- or L-) with a neutral side chain are selected from the following group: Ala, Asn, Cys, Gln, Gly, Ile, Leu, Met, Pro, Phe, Sar, Ser, hSer, Thr, Trp, Tyr and Val.

The compounds of formula II can be prepared as described in the examples given below, and by methods which are generally known in peptide chemistry. Briefly, compounds of formula II can be prepared entirely by conventional peptide synthesis is solution, entirely by solid phase peptide synthesis or by a combination of conventional and solid phase peptide synthesis.

Common to the synthesis of the preferred compounds of formula II is the protection of the labile side chain groups, which will prevent an undesired chemical reaction from occurring at these sites until the groups are ultimately removed. Such a protection is designated a permanent protection. Also common is the protection of an α-amino group in an amino acid or a peptide fragment while that entity reacts at the carboxyl group,

followed by the selective removal of the α-amino protecting group to allow subsequent reaction to take place at that location. Such a protection is designated a temporary protection. Accordingly, it is common that as a step in the synthesis an intermediate peptide fragment is produced which includes each of the amino acid residues located in its desired position in the peptide chain with permanent protecting groups linked to the amino acid side chain where appropriate, and with the carboxyl group of the C-terminus protected with a group that can be removed selectively without interfering with the permanent protection, but at the same time being stable under the conditions used to remove the α-amino protecting group. Such a protection is designated a semipermanent protection.

Compounds of the general formula II can be built up by methods of conventional peptide synthesis in solution by analogy with procedures described by M. Bodanszky in Principles of Peptide Synthesis (Springer-Verlag 1984) and in The Peptides 1-5, Ed. E. Gross and J. Meienhofer (Academic Press 1979) using conveniently protected ANP-fragments.

The peptides according to this application are preferably prepared by solid phase peptide synthesis in a manner analogous to the procedures described by J.M. Stewart and J.D. Young in Solid Phase Peptide Synthesis, 2nd edition (1984), using the methodology described in Examples 1-4 below. Among the classes of temporary protecting groups for this procedure are selected tert-butoxycarbonyl (hereinafter designated Boc) and among the classes of permanent protection groups, benzyl (hereinafter designated Bzl), 2-bromobenzyloxycarbonyl (hereinafter designated Br-Z), 4-methoxybenzyl (hereinafter designated Mob), 4-methylbenzyl (hereinafter designated MeB), 4-toluenesulfonyl (hereinafter designated Tos), mesitylene-2-sulfonyl (hereinafter designated Mts) and cyclohexyl (hereinafter designated cHex). The resin utilized is of the so-called "PAM" polystyrene type as described in J.Org.Chem. 43 (1978), 2845-52, or the so-called "MBHA" polystyrene type as described in Peptides 2 (1981), 45-50.

The synthesis is performed essentially as described in Example 1, using an Applied Biosystems 430 A automatic peptide synthesizer. The amino acids are preferably activated as symmetrical anhydrides or as 1-hydroxybenzotriazole (hereinafter designated HOBt) esters. Each protected amino acid is introduced into the solid phase reactor in about a two to fourfold excess, and the coupling is mainly carried out in N,N-dimethylformamide (hereinafter designated DMF).

After the desired amino acid sequence has been completed, the intermediate peptide is removed from the resin support by treatment with a reagent, such as liquid hydrogen fluoride (HF) either by the "low" procedure, the "low-high" procedure or by HF/anisole (9/1) as described by J.M. Stewart and J.D. Young in Solid Phase Peptide Synthesis, 2nd edition (1984). At the same time these procedures remove all side chain protecting groups leaving the peptide in its linear form. In the case of a cystine containing peptide, the cyclic form of the peptide is obtained by oxidizing the linear form in a ferricyanide solution, preferably as described in Biochem.Biophys.Res.Comm. 131 (1985), 1056 - 62, or in accordance with other known procedures. In case the C-terminal residue is to be linked to the N-terminal amino group or to an alkyl or dialkyl amine by an amide bond, the side chain protection groups and the reaction conditions during removal of the peptide from the resin are selected to keep the permanent protection on the reactive groups during the formation of the peptide bond by the carbodiimide/HOBt procedure. These protection groups are subsequently cleaved with liquid HF.

The crude peptide is subjected to semipreparative HPLC purification as described in Example 1 below.

Compounds of this invention containing only amino acids, which can be coded for by the genetic code, can be produced by expression of recombinant DNA constructs. Such production can be desirable to provide large quantities or alternative embodiments of such compounds.

More particularly, modifications in the amino acid sequence of the various forms of pre-proANP, proANP and ANP compounds can be effected by various changes in the nucleotide sequence of the cloned structural gene used to direct the synthesis of compounds of formula II. Included within such modification of the DNA sequence are the replacement of various codons with other codons which, due to the degeneracy of the genetic code, direct the synthesis of the same amino acid.

In addition, by codon substitution, one or more amino acid residues can be replaced by functionally equivalent residues.

Whenever two cystein residues are present in a molecule according to the invention they are in oxidised form, i.e. forming an intramolecular disulphide bridge.

Compounds of this invention have natriuretic and diuretic activity in the intact mammal and in the kidney isolated from a mammal. Furthermore, compounds of this invention possess vasorelaxant activity and inhibit the release of aldosterone.

Compounds of this invention can find use in numerous therapeutic applications such as, for example, inducing natriuresis, diuresis and vasodilatation. Thus these compounds can find use as therapeutic agents in the treatment of various oedematous states such as, for example, congestive heart failure, nephrotic syndrome and hepatic cirrhosis, in addition to hypertension and renal failure due to ineffective renal perfusion or reduced glomerular filtration rate.

Compounds of formula II can be administered to humans and to animals, e.g. domestic animals, in a manner similar to other therapeutic agents, that is in a pharmaceutically acceptable carrier. In general the daily dosage of compounds of formula II will range from about 0.01 to 100 μg/kg, more usually from about 0.1 to 10 μg/kg of the host body weight. Alternatively, dosages within these ranges can be administered by constant infusion over an extended period of time, usually exceeding 24 hours, until the desired therapeutic benefits have been obtained.

Compounds of formula II can be administered neat, as mixtures with other pharmaceutically acceptable active or inactive materials, for example carriers such as water or saline. Compounds of formula II can be administered nasally, sublingually or parenterally, for example, by injection. Compounds of formula II can be injected subcutaneously, intravenously or intramuscularly.

Compounds of formula II are desirably administered in a pharmacologically effective amount, optionally in the form of pharmaceutically acceptable salts such as acid addition salts with pharmaceutically acceptable acids. Such salts can include, for example, the hydrochloride, hydrobromide, phosphate, sulphate, acetate, benzoate, or maleate.

Compounds of this invention can also be used for preparing antisera for use in immunoassays employing labelled reagents, usually antibodies.

Test for vasodilating activity (in vitro)

A rabbit (NZ white) was killed by a blow to the neck and exsanguination. The left renal artery was removed, cut helically and immediately suspended in an organ bath containing Krebs' solution kept at 37° C and aerated with 5% carbon dioxide in 95% oxygen. Contractions of the preparation were recorded isotonically, the tension being 860 mg.

After the preparation had been mounted in the organ bath, a relaxation occured. When this relaxation had faded, doses of noradrenaline were added to the bath until the response was stable. During this period of time, a maximal (100%) contraction should be achieved at least once. Before each dose of noradrenaline, a period of full relaxation lasting for at least 5 minutes was allowed to pass.

In order to standardize the test so that the activity of different atrial natriuretic peptides can be characterized and compared in a uniform way, a concentration of noradrenaline was found which leads to a contraction of 30 to 40% of the maximal contraction. Against this fixed noradrenaline concentration, the peptides were tested in two or three concentrations and the concentration-response curves were compared with a similar curve obtained with ANP(5-28).

On the basis of the experimental data the potency of the test compounds relative to ANP(5-28) was calculated. The results are listed in Table 1.

Table 1

| Compound | Potency relative to ANP(5-28) |
|---|---|
| [Ac-Cys$^{7}$,Cys$^{23}$-NH$_2$]-ANP(7-23)(reference) | 0.002 |
| [Ac-Cys$^{7}$,Arg$^{20}$,Phe$^{21}$,Cys$^{23}$-NH$_2$]-ANP(7-23) | 0.05 |
| [Ac-Cys$^{7}$,D-Arg$^{20}$,Phe$^{21}$,Cys$^{23}$-NH$_2$]-ANP(7-23) | 0.2 |
| [Ac-Cys$^{7}$,D-Phe$^{9}$,Arg$^{20}$,Phe$^{21}$,Cys$^{23}$-NH$_2$]-ANP(7-23) | 0.03 |
| [Ac-Cys$^{7}$,Arg$^{17}$,Arg$^{20}$,Phe$^{21}$,Cys$^{23}$-NH$_2$]-ANP(7-23) | 0.3 |
| [Ac-Cys$^{7}$,Arg$^{20}$,Cys$^{23}$-NH$_2$]-ANP(7-23) | 0.03 |
| [Ac-Cys$^{7}$,Arg$^{17}$,D-Arg$^{20}$,Phe$^{21}$,Cys$^{23}$-NH$_2$]-ANP(7-23) | 1.8 |
| [Ac-Cys$^{7}$,Arg$^{17}$,D-Arg$^{20}$,Cys$^{23}$-NH$_2$]-ANP(7-23) | 0.7 |
| [Ac-Cys$^{7}$,Gly$^{13}$,Arg$^{17}$,D-Arg$^{20}$,Phe$^{21}$,Cys$^{23}$-NH$_2$]-ANP(7-23) | 0.2 |
| cyclo(8,22)[Phe$^{8}$,Arg$^{17}$,D-Arg$^{20}$,Phe$^{21}$,Gly$^{22}$]-ANP(8-22) | 0.01 |
| cyclo(8,22)[Phe$^{8}$,Arg$^{17}$,D-Arg$^{20}$,Phe$^{21}$,8-aminooctanoyl$^{22}$]-ANP(8-22) | 3 |
| Ac-Cys-Phe-Gly-Gly-Arg-Ile-Asp-Arg-Ile-Arg-Gly-Arg-D-Phe-Gly-Cys-NH$_2$ | 0.2 |
| Ac-Cys-Phe-Gly-Gly-Arg-Ile-Asp-Arg-Ile-Gly-Arg-Gly-D-Arg-Phe-D-Cys-NH$_2$ | 0.05 |
| cyclo(8,22)[Phe$^{8}$,Arg$^{17}$,D-Arg$^{20}$,Phe$^{21}$,Sar$^{22}$]-ANP(8-22) | 0.1 |
| [Ac-Cys$^{7}$,D-Ala$^{9}$,Arg$^{17}$,Leu$^{18}$,D-Arg$^{20}$,Phe$^{21}$,D-Ala$^{22}$,Cys$^{23}$-NH$_2$]-ANP(7-23) | 7 |
| cyclo(8,22)[Phe$^{8}$,D-Ala$^{9}$,Arg$^{17}$,Leu$^{18}$,D-Arg$^{20}$,Phe$^{21}$,8-aminooctanoyl$^{22}$]-ANP(8-22) | 3 |
| Des(Gln$^{18}$,Ser$^{19}$)[Ac-D-Cys$^{7}$,Arg$^{17}$,D-Arg$^{20}$,Phe$^{21}$,Cys$^{22}$-NH$_2$]-ANP(7-22) | 0.07 |
| Des(Gln$^{18}$,Ser$^{19}$)[Ac-Cys$^{7}$,Arg$^{17}$,D-Arg$^{20}$,Phe$^{21}$,Cys$^{23}$-NH$_2$]-ANP(7-23) | 0.07 |
| [Ac-D-Cys$^{7}$,Arg$^{17}$,D-Arg$^{20}$,Phe$^{21}$,Cys$^{22}$-NH$_2$]-ANP(7-22) | 10 |

Example 1

[Ac-$Cys^7$, D-$Phe^9$, $Arg^{20}$, $Phe^{21}$, $Cys^{23}$-$NH_2$]-ANP(7-23) was prepared entirely by solid phase peptide synthesis.

Assembly of the protected [Ac-$Cys^7$,D-$Phe^9$, $Arg^{20}$,$Phe^{21}$,$Cys^{23}$-$NH_2$]-ANP(7-23) sequence on the resin was performed as described below. The resin utilized was of the so-called "MBHA" type which is described in G.R. Matsueda and J.M. Stewart: Peptides 2 (1981), 45 - 50 and is obtained from Applied Biosystems, U.S.A. (part No. 400229).

The following commercially available amino acid derivatives were applied: Boc-Arg(Tos)-OH, Boc-Phe-OH, Boc-D-Phe-OH, Boc-Asp(OBzl)-OH, Boc-Ser(Bzl)-OH, Boc-Cys(Mob)-OH, Boc-Gly-OH, Boc-Gln-OH, Boc-Ile-OH, and Boc-Ala-OH.

The assembly of the peptide chain was done successively by an automatic peptide synthesizer (Applied Biosystems United States, model 430A).

The incorporation of an amino acid consisted essentially of the following operations:

1) Removal of the Boc-protecting group from the previously attached amino acid group by treatment with trifluoroacetic acid/$CH_2Cl_2$ (50/50) for 2 minutes and then with trifluoroacetic acid/$CH_2Cl_2$ (70/30) for 20 minutes at room temperature followed by washing with $CH_2Cl_2$.

2) Conversion of the protonated amino group to its unprotonated form by treatment with diisopropylethylamine/DMF (1/6) followed by washing with DMF.

3) Coupling of the appropriately protected amino acid derivative either as the symmetrical anhydride (2 equivalents) or as the HOBt ester (4 equivalents) to the resin bound peptide at room temperature in a process lasting for 16 - 240 minutes followed by washing with DMF and subsequently with $CH_2Cl_2$.

All the amino acid derivatives were coupled as symmetrical anhydrides except for Boc-Gln-OH and Boc-Arg(Tos)-OH which were coupled as their HOBt esters. In order to improve the yield, the coupling can be repeated in the cases of Boc-Gln-OH and Boc-Arg(Tos)-OH.

The HOBt esters were prepared in the automatic peptide synthesizer by a reaction of the pertinent amino acid derivatives, HOBt (1 equivalent) and N,N′-dicyclohexylcarbodiimide (hereinafter designated DCC) (1 equivalent) in DMF for 25 minutes at room temperature. The resulting solutions were filtered and used directly for coupling.

The symmetrical anhydrides were prepared in the automatic peptide synthesizer by a reaction of the amino acid derivative with DCC (0.5 equivalents) in $CH_2Cl_2$ for 6 minutes at room temperature. After filtration, the $CH_2Cl_2$ was evaporated and replaced by DMF and the resulting solution was used for coupling.

After the construction of the peptide chain was completed, and terminal Boc group was removed as described above and the resin linked peptide was $N^{\alpha}$-acetylated with acetic anhydride, washed with $CH_2Cl_2$ and dried. 218 mg of the peptide resin was subjected to a cleavage and deprotection procedure using a mixture of hydrogen fluoride and anisole as described by J.M. Stewart and J.D. Young in Solid Phase Synthesis, 2nd edition (1984). After evaporation of the hydrogen fluoride the liberated peptide was precipitated and washed several times with tert-butyl methyl ether. Then the precipitate was extracted with 8 ml of 50% acetic acid and the extract was subjected to gel filtration in 2% acetic acid containing 15% of 2-propanol on a column of Sephadex G-25. The peptide containing fractions were collected and 12.1 mg of dithiothreitol was added in order to cleave optional disulphide bonds in the product. The pH of the solution was brought to 9.5 (ammonium hydroxide solution) and the solution was left for 30 minutes at room temperature. Then the pH value was adjusted to 8.0 (acetic acid) and the resulting mixture was diluted to 250 ml (water). A solution of 172 mg of potassium ferricyanide in 25 ml of water was added with stirring during 2 hours. The pH value was then lowered further to 5.0 (acetic acid) and the solution was treated with 1.5 g of QAE-Sephadex A-25 ion exchanger (on $Cl^-$ form) for 0.5 hours. The ion exchanger was removed by filtration and the resulting colourless solution was lyophilized.

Purification

32 mg of the crude product (containing approximately 20% of the desired peptide) was dissolved in 5% acetic acid (200 ml). After sonication, the solution was filtered (Millex-GV 0.22 μm) and 80 ml of the filtrate were subjected to chromatography on a reversed phase column (16 x 250 mm RP-18, 7 μm). The column was eluted at 6 ml/min. with an acetonitrile/0.2 M ammonium acetate mixture (pH 3.3). The initial concentration of $CH_3CN$ was 25% and it was increased by a linear gradient to 40% during 48 minutes. Fractions containing the product were collected and combined. To the combined fractions was added an equal amount of water and the solution obtained was concentrated to half volume (rotary evaporator) and diluted with water to 50 ml and then the product was lyophilized at -20° C. The product was characterized by amino acid analysis and HPLC analysis.

For the HPLC analysis a 4.6 mm x 25 cm 5 μ VYDAC 218TP54 column was used. The column was eluted at 1 ml/min. with an acetonitrile/0.1 M $(NH_4)_2SO_4$ mixture. The initial concentration of $CH_3CN$ was 15% and it was increased by a linear gradient to 35% during 25 minutes (condition A). Retention time for the desired product: 22.93 minutes.

The amino acid analysis was in agreement with the intended structure of the product.

Example 2

The following compounds were prepared by procedures similar to those described in Example 1 and were subjected to HPLC analysis under the same conditions. For all the compounds, the amino acid analysis was in agreement with the intended structure.

| Compound | Retention time (min) |
|---|---|
| [Ac-Cys$^{7}$,Arg$^{20}$,Phe$^{21}$,Cys$^{23}$-$NH_2$]-ANP(7-23) | 15,08 |
| [Ac-Cys$^{7}$,D-Arg$^{20}$,Phe$^{21}$,Cys$^{23}$-$NH_2$]-ANP(7-23) | 16,13 |
| [Ac-Cys$^{7}$,Arg$^{17}$,Arg$^{20}$,Phe$^{21}$,Cys$^{23}$-$NH_2$]-ANP(7-23) | 13,72 |
| [Ac-Cys$^{7}$,Arg$^{20}$,Cys$^{23}$-$NH_2$]-ANP(7-23) | 13,72 |
| [Ac-Cys$^{7}$,Arg$^{17}$,D-Arg$^{20}$,Phe$^{21}$,Cys$^{23}$-$NH_2$]ANP(7-23) | 12,46 |
| [Ac-Cys$^{7}$,Arg$^{17}$,D-Arg$^{20}$,Cys$^{23}$-$NH_2$]-ANP(7-23) | 12,84 |
| [Ac-Cys$^{7}$,Gly$^{13}$,Arg$^{17}$,D-Arg$^{20}$,Phe$^{21}$,Cys$^{23}$-$NH_2$]-ANP(7-23) | 13,40 |
| Ac-Cys-Phe-Gly-Gly-Arg-Ile-Asp-Arg-Ile-Arg-Gly-Arg-D-Phe-Gly-Cys-$NH_2$ | 11,71 |
| Ac-Cys-Phe-Gly-Gly-Arg-Ile-Asp-Arg-Ile-Gly-Arg-Gly-D-Arg-Phe-D-Cys-$NH_2$ | 13,73 |
| [Ac-Cys$^{7}$,D-Ala$^{9}$,Arg$^{17}$,Leu$^{18}$,D-Arg$^{20}$,Phe$^{21}$,D-Ala$^{22}$-Cys$^{23}$-$NH_2$]-ANP(7-23) | 19,50 |
| Des(Gln$^{18}$,Ser$^{19}$)[Ac-D-Cys$^{7}$,Arg$^{17}$,D-Arg$^{20}$,Phe$^{21}$,Cys$^{22}$-$NH_2$]-ANP(7-22) | 15,69 |
| Des(Gln$^{18}$,Ser$^{19}$)[Ac-Cys,Arg$^{17}$,D-Arg$^{20}$,Phe$^{21}$,Cys$^{23}$-$NH_2$]-ANP(7-23) | 13,35 |
| [Ac-D-Cys$^{7}$,Arg$^{17}$,D-Arg$^{20}$,Phe$^{21}$,Cys$^{22}$-$NH_2$]-ANP(7-22) | 15.17 |
| [3-Thiopropionyl$^{7}$,D-Ala$^{9}$,Arg$^{17}$,Leu$^{18}$,D-Arg$^{20}$,Phe$^{21}$,D-Ala$^{22}$,Cys$^{23}$-$NH_2$]ANP(7-23) | 20,45 |
| [Ac-D-Cys$^{7}$,D-Ala$^{9}$,Arg$^{17}$,Leu$^{18}$,D-Arg$^{20}$,Phe$^{21}$,Cys$^{22}$-$NH_2$]-ANP(7-22) | 19,61 |
| [3-Thiopropionyl$^{7}$,Arg$^{17}$,D-Arg$^{20}$,Phe$^{21}$,Cys$^{22}$-$NH_2$]-ANP(7-22) | 16,28 |
| [3-Thiopropionyl$^{7}$,D-Ala$^{9}$,Arg$^{17}$,Leu$^{18}$,D-Arg$^{20}$,Phe$^{21}$,Cys$^{22}$-$NH_2$]-ANP(7-22) | 20,56 |

Example 3

Cyclo(8,22)[Phe$^{8}$,Arg$^{17}$,D-Arg$^{20}$,Phe$^{21}$,Sar$^{22}$]-ANP(8-22) was prepared by a combination of solid phase peptide synthesis and synthesis in solution.

Assembly of the protected linear peptide sequence was performed as described in Example 1, except for the following operations:

The resin utilized was a "PAM" polystyrene type, described in J. Org. Chem. 43 (1978), 2845-52. The actual type used in this synthesis was a Boc-Gly-PAM-resin obtained from Applied Biosystems, U.S.A. (Part No. 400097).

The following commercially available amino acid derivatives were used: Boc-Phe, Boc-Ser-OH, Boc-D-Arg(Tos)-OH, Boc-Ser(Bzl)-OH, Boc-Gln-OH, Boc-Arg(Tos)-OH, Boc-Gly-OH, Boc-Ile-OH, Boc-Asp-(OcHex)-OH, Boc-Ala-OH and Boc-Sar-OH.

The synthesis of the peptide was started with 0.548 grams of Boc-$Gly^9$-PAM-resin. After construction of the linear peptide was completed, the Boc group attached to the amino group of $Gly^{10}$ was removed, leaving this amino group as N-terminal of the peptide prior to cyclization. 1.29 grams of protected peptide-resin were obtained.

310 mg of peptide resin were subjected to the "Low" procedure, described by J.P. Tam, W.F. Heath and R.B. Merrifield in J.Am. Chem. Soc., 105 (1983) 6442-55, in order to cleave the Bzl protecting groups from the protein and in order to cleave the bond between the peptide and the resin.

After the cleavage the hydrogen fluoride and dimethyl sulfide was evaporated and the liberated, partially protected peptide was precipitated and washed several times with diethyl ether and dried. To the dried material was added 15 ml of 70% 2-PrOH/0.03M HCl and after sonication for 30 min. undissolved material was filtered off. The filtrate was diluted with water (25 ml) until precipitation of the peptide was achieved. The precipitate was isolated by centrifugation and after washing several times with 14% 2-PrOH/0.03M HCl, 55 mg of the partially protected peptide was obtained.

The product isolated was dissolved in 75 ml DMF, and 14.8 µl N-methylmorpholine (6 equivalents), 14.8 mg HOBt (4.4 equivalents) and 16.8 mg N-(dimethylaminopropyl)-N′-ethylcarbodiimide (4 equivalents) was added. The solution so obtained was left with gentle stirring for 72 hours at room temperature.

Then the reaction mixture was diluted with water (95 ml) and loaded on a $C_{18}$ cartridge (Seppaks, Waters Associates, U.S.A.). The cartridge was eluted with 10 ml of 70% 2-PrOH/0.03M HCl and the eluate was evaporated to dryness.

The final deprotection of the cyclized peptide was performed in a mixture of hydrogen fluoride and m-cresol as described in the previously mentioned "High" procedure by J.P. Tam et al.

Purification of the final product was performed by the procedure described in Example 1.

For this compound a retention time of 13.44 min. was found using the analytical HPLC system described in Example 1.

The amino acid analysis was in agreement with the intended structure of the product.

Example 4

The following compounds have been prepared by a procedure similar to the one described in Example 3.

| Compound | Retention time(min) |
|---|---|
| cyclo(8,22)[$Phe^8$,D-$Ala^9$,$Arg^{17}$,$Leu^{18}$,D-$Arg^{20}$,$Phe^{21}$, 8-aminooctanoyl$^{22}$]-ANP(8-22) | 23,25 |
| cyclo(8,22)[$Phe^8$,$Arg^{17}$,D-$Arg^{20}$,$Phe^{21}$,$Gly^{22}$]-ANP(8-22) | 12,31 |
| cyclo(8,22)[$Phe^8$,$Arg^{17}$,D-$Arg^{20}$,$Phe^{21}$,8-aminooctanoyl$^{22}$]-ANP(8-22) | 18,56 |

Retention times were found using the analytical HPLC system described in Example 1.

The features disclosed in the foregoing description and in the following claims may, both separately and in any combination thereof, be material for realizing the invention in diverse forms thereof.

**Claims**

1. Compound of the general formula II

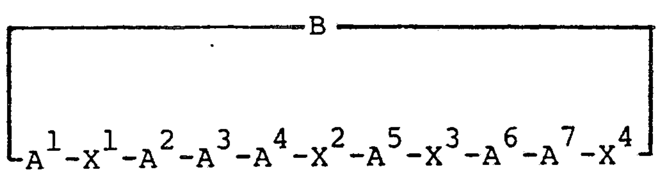

(II)

wherein
$A^1$ is Phe or D-Phe;
$A^2$ is Asp, D-Asp, Glu or Gly;
$A^3$ is Arg or hArg;
$A^4$ is Ile, Leu or Val;
$A^5$ is Arg, D-Arg, hArg, another amino acid residue with a basic side chain or Ala;
$A^6$ is D-Arg, Arg or another amino acid residue in the D-or L-configuration with a basic side chain or Ala;
$A^7$ is Leu, Phe or another amino acid residue with an aromatic side chain;
$X^1$ is a spacer of the formula

$$-(A^a)_{n_1}-(A^b)_{n_2}-(A^c)_{n_3}-$$

where $A^a$,$A^b$, $A^c$ and $A^d$ each are amino acid residues (D- or L-) with a neutral or a basic side chain other than the sarcosine residue and $n_1$, $n_2$, $n_3$ and $n_4$ independently are zero or one;
$X^2$ is a spacer of the formula

$$(A^e)_{n_5}-$$

where $A^e$ is an amino acid residue with a neutral side chain other than the sarcosine residue and $n_5$ is zero or one;
$X^3$ is a spacer of the formula

$$-(A^f)_{n_6}-(A^g)_{n_7}-$$

where $A^f$ and $A^g$ each are amino acid residues with a neutral side chain other than the sarcosine residue and $n_6$ and $n_7$ independently are zero or one;
$X^4$ is a spacer of the formula

$$-(A^h)_{n_8}-(A^k)_{n_9}-$$

where $A^h$ and $A^k$ each are amino acid residues in the D- or L-configuration with a neutral side chain or in the case of $A^h$: 8-aminooctanoic acid and $n_8$ and $n_9$ independently are zero or one;
B is either a peptide bond connecting the N-terminus of $A^1$ with the C-terminus of $X^4$ or B is a substructure of the general formula III

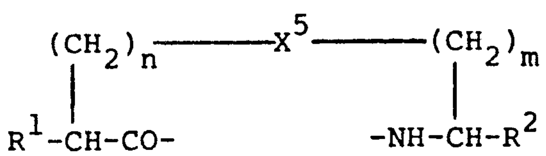

(III)

which is oriented so that the -CO- group is linked to the amino group of $A^1$ and the -NH- group is linked to the carboxyl group of $X^4$ and in which
$R^1$ is hydrogen, amino or lower acylamino
$R^2$ is hydrogen, carboxyl, or carbamoyl
$X^5$ is a carbon-carbon bond or a group selected from -S-S-, -CO-NH- or -NH-CO-;
n and m independently are 0, 1, 2, 3 or 4 with the proviso that the total number of chain members in the part of B which connects the N-terminus of $A^1$ with the C-terminus of $X^4$ is from 4 up to 9, and salts thereof.

2. Compound according to Claim 1, wherein $A^1$ is Phe or D-Phe, preferably Phe.

3. Compound according to any of the preceding claims wherein $A^2$ is Asp, D-Asp, Glu- or Gly, preferably Asp.

4. Compound according to any of the preceding claims wherein $A^3$ is Arg or hArg, preferably Arg.

5. Compound according to any of the preceding claims wherein $A^4$ is Ile, Leu or Val, preferably Ile.

6. Compound according to any of the preceding claims wherein $A^5$ is Arg, D-Arg, hArg, another amino acid residue with a basic side chain or Ala, preferably Arg.

26. Compound according to any of the preceding claims wherein $A^6$ is D-Arg, Arg or another amino acid residue in the D- or L-configuration with a basic side chain, preferably D-Arg.

8. Compound according to any of the preceding claims wherein $A^7$ is Leu, Phe or another amino acid residue with an aromatic side chain; preferably Phe.

9. Compound according to any of the preceding claims wherein $X^1$ is a spacer of the general formula

$$-(A^a)_{n_1}-(A^b)_{n_2}-(A^c)_{n_3}-(A^d)_{n_4},$$

wherein $A^a$, $A^b$, $A^c$ and $A^d$ each are amino acid residues (D- or L-) with a neutral or basic side chain other than the sarcosine residue and $n_1$, $n_2$, $n_3$ and $n_4$ independently are zero or one.

10. Compound according to any of the preceding claims wherein $n_1$, $n_2$, $n_3$ and $n_4$ are all one.

11. Compound according to any of the preceding claims wherein $A^a$ is an amino acid residue with a neutral side chain, preferably Gly, D-Ala or D-Phe.

12. Compound according to any of the preceding claims wherein $A^b$ is Gly.

13. Compound according to any of the preceding claims wherein $A^c$ is Arg.

14. Compound according to any of the preceding claims wherein $A^d$ is Ile.

15. Compound according to any of the preceding claims wherein $X^2$ is a spacer of the general formula

$$(A^e)_{n_5}-$$

where $A^e$ is an amino acid residue with a neutral side chain other than the sarcosine residue and $n_5$ is zero or one.

16. Compound according to any of the preceding claims wherein $n_5$ is one.

17. Compound according to claim 16 wherein $A^e$ is Ala or Gly.

18. Compound according to any of the claims 1 through 15 wherein $n_5$ is zero.

19. Compound according to any of the preceding claims wherein $X^3$ is a spacer of the general formula

$$-(A^f)_{n_6}-(A^g)_{n_7}-$$

where $A^f$ and $A^g$ each are amino acid residues with a neutral side chain other than the sarcosine residue and $n_6$ and $n_7$ independently are zero or one.

20. Compound according to any of the preceding claims wherein $n_6$ is one.

21. Compound according to Claim 20 wherein $A^f$ is an amino acid residue with a neutral side chain, preferably Gln, Leu, Ala or Gly.

22. Compound according to any of the claims 1 through 19 wherein $n_6$ is zero.

23. Compound according to any of the preceding claims wherein $n_7$ is one.

24. Compound according to Claim 23 wherein $A^g$ is an amino acid residue with a neutral side chain, preferably Ser, Ala or Gly, more preferred Ser.

25. Compound according to any of the claims 1 through 22 wherein $n_7$ is zero.

26. Compound according to any of the preceding claims wherein $X^4$ is a spacer of the general formula

$$-(A^h)_{n_8}-(A^k)_{n_9}-$$

where $A^h$ and $A^k$ are amino acid residues with a neutral side chain and $n_8$ and $n_9$ independently are zero or one.

27. Compound according to any of the preceding claims wherein $n_8$ is one.

28. Compound according to claim 27 wherein $A^h$ is an amino acid residue with a neutral side chain, preferably Gly, D-Ala, Ala or Sar.

29. Compound according to Claim 27 wherein $A^h$ is 8-aminooctanoic acid.

30. Compound according to any of the claims 1 through 26 wherein $n_8$ is zero.

31. Compound according to any of the preceding claims wherein $n_9$ is zero.

32. Compound according to any of the preceding claims wherein B is a substructure of the general formula III

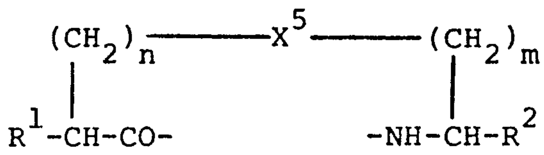

(III)

which is oriented so that the -CO- group is linked to the N-terminus of $A^1$ and the -NH- group is linked to the C-terminus of $X^4$ and in which
$R^1$ is hydrogen, amino or lower acylamido
$R^2$ is hydrogen, carboxyl or carbamoyl
$X^5$ is a carbon-carbon bond or a group selected from -S-S-, -CO-NH- or -NH-CO- and
n and m independently are 0, 1, 2, 3 or 4 with the proviso that the total number of chain members in the part of B which connects the N-terminus of $A^1$ with the C-terminus of $X^4$ is from 4 up to 9.

33. Compound according to any of the preceding claims wherein $R^1$ is hydrogen.
34. Compound according to any of the claims 1 through 32 wherein $R^1$ is lower acylamido, preferably acetamido.
35. Compound acc ording to any of the preceding claims wherein $R^2$ is carbamoyl.
36. Compound according to any of the preceding claims wherein $X^5$ is a disulphide bridge (-S-S-).
37. Compound according to any of the preceding claims wherein n is 1.
38. Compound according to any of the preceding claims wherein m is 1.
39. Compound according to any of the claims 1 through 31 wherein B is a peptide bond connecting the amino group of $A^1$ with the carboxyl group of $X^4$.
40. A process for preparing the compounds according to claim 1 characterized in removing protection groups(s), including polymer linked anchor groups from a protected precursor for a compound of formula II by an acidolytic reaction in which the protected compound precursor is treated with an acid such as hydrogen fluoride, trifluoroacetic acid, trifluoromethanesulfonic acid, methanesulfonic acid, or mixtures thereof in the presence of proper scavengers and in case the precursor is linear followed by a cyclisation reaction which is either the formation of a disulphide bridge using oxidizing agents such as iodine or potassium ferricyanide or a peptide bond forming reaction using a peptide coupling reagent such as N,N′-dicyclohexylcarbodiimide in presence of additives such as 1-hydroxybenzotriazole, or diphenyl phosphoryl azide and optionally converting the product to an acid addition salt thereof.
41. A pharmaceutical composition comprising one or more compounds or salts according to any one or more of Claims 1 to 39 and one or more pharmaceutically acceptable active or inactive materials.
42. The use of one or more compounds or salts according to any one or more of Claims 1 to 39 for preparing antisera for use in immunoassays employing labelled reagents, usually antibodies.